# EUROPEAN PATENT SPECIFICATION

(11) **EP 2 591 781 B1**
(45) Date of publication and mention of the grant of the patent: **27.09.2017**
(21) Application number: 11803699.5
(22) Date of filing: 08.07.2011
(51) Int. Cl.: A61K 31/426, A61K 9/20, A61K 47/10, A61K 47/32, A61K 9/00, A61K 9/50, A61K 31/425

(54) **PARTICLE COATING PREPARATION**
PRÄPARAT MIT PARTIKELBESCHICHTUNG
PRÉPARATION D'UN REVÊTEMENT DE PARTICULES

(30) Priority: 09.07.2010 JP 2010156874
(43) Date of publication of application: 15.05.2013
(73) Proprietor: Teijin Pharma Limited, Tokyo 100-0013 (JP)
(72) Inventor: NAKAMURA, Kazuhiro, Iwakuni-shi, Yamaguchi 740-0014 (JP); OGAWA, Teppei, Iwakuni-shi, Yamaguchi 740-0014 (JP); AKUTAGAWA, Tomoya, Iwakuni-shi, Yamaguchi 740-0014 (JP)
(74) Representative: Cockerton, Bruce Roger
(86) International application number: PCT/JP2011/065721
(87) International publication number: WO 2012/005365

(56) References cited:
- EP-A1- 1 915 988
- WO-A1-03/043661
- WO-A1-2011/159745
- WO-A2-99/59544
- JP-A- 2000 281 564
- JP-A- 2007 153 887
- JP-A- 2008 050 324
- JP-A- 2009 515 871

## Description

### [TECHNICAL FIELD]

The present invention relates to an intraorally rapidly disintegrating tablet comprising 2-(3-cyano-4-isobutyloxyphenyl)-4-methyl-5-thiazolecarboxylic acid (hereinafter sometimes referred to as "Compound I") as an active ingredient, which is a formulation that causes virtually no irritating sensation in the oral cavity or pharynx, and retains good dissolution properties even when it is stored under high temperature/high humidity conditions. More specifically, the present invention relates to an intraorally rapidly disintegrating tablet comprising a particle of Compound I coated with a layer containing a methacrylic acid copolymer and further overcoated with a water-soluble saccharide.

### [BACKGROUND ART]

Intraorally rapidly disintegrating tablets disintegrate rapidly in the oral cavity, and consequently have been drawing attention as a dosage form to enhance patient's compliance by improving easiness of taking drugs, and various intraorally rapidly disintegrating tablets are known. Rapid disintegration in the oral cavity, however, works adversely in terms of sense of taste, such as bitterness and the like derived from drugs, and, therefore, masking the bitterness and the like is often a problem. Various techniques for masking the bitterness and the like in the oral cavity are known in the area of intraorally rapidly disintegrating tablets, including, for example, methods by mixing a combination of a menthol and a sweetener (Patent Document 1), by mixing a combination of an essential oil and a high-intensity sweetener (Patent Document 2), by mixing calcium lactate as a masking agent (Patent Document 3), or the like. In addition, there are also methods by coating a drug itself, or a granule containing a drug, with a sustained-release polymer, a gastrosoluble polymer, or an enteric polymer, or the like (Patent Document 4).

On the other hand, there is concern that Compound I, when formulated into an intraorally rapidly disintegrating tablet, might leave within the oral cavity or pharynx a distinctive irritating sensation caused by Compound I. However, when the above techniques for masking bitterness and the like were applied to Compound I, the masking was not sufficient by means of mixing a sweetener or a masking agent such as calcium lactate as in Patent Documents 1 to 3. In addition, although increasing the amount of coating would make it possible to sufficiently mask the irritation of a drug by means of coating a sustained-release polymer or gastrosoluble polymer as in Patent Document 4, the dissolution of Compound I from formulations would be extremely suppressed, which raised concern about the influence on medicinal effects.

Furthermore, although the method by coating an enteric polymer (Patent Document 5) resulted in good masking properties and showed no influence on the dissolution of Compound I from formulations, it has been found that there exist the problems of poor dissolution of Compound I from formulations or poor disintegration of formulations in the oral cavity after storage, especially after storage under high temperature/high humidity conditions.

In addition, although Patent Document 6 discloses a technique in which a particle coated with an enteric polymer, including methacrylic acid copolymers, is further coated with a water-soluble sugar alcohol, active ingredients are limited to acid-labile benzimidazol compounds or salts thereof, and the effect of the water-soluble sugar alcohol coating consists in increasing the hardness of intraorally rapidly disintegrating tablets .

### [RELATED ART DOCUMENTS]

### [PATENT DOCUMENTS]

[Patent Document 1] Japanese Patent Laid-Open Publication No. 2000-159691
[Patent Document 2] Japanese Patent Laid-Open Publication No. 2001-072578
[Patent Document 3] Japanese Patent Laid-Open Publication No. 2008-094837
[Patent Document 4] Japanese Patent Laid-Open Publication No. 2005-060309
[Patent Document 5] Japanese Patent Laid-Open Publication No. 2005-023058
[Patent Document 6] Japanese Patent Laid-Open Publication No. 2000-281564

EP 1 915 988 discloses preparation of a tablet wherein "granules made of febuxostat, lactose, partially gelatinized starch and hydroxypropylcellulose and granules made of lactose, partially gelatinized starch and hydroxypropylcellulose were mixed with croscarmellose sodium and magnesium stearate, respectively".

### [SUMMARY OF THE INVENTION]

### [PROBLEMS TO BE SOLVED BY THE INVENTION]

An object of the present invention is to provide an intraorally rapidly disintegrating tablet comprising compound I as an active ingredient, which is a preparation that causes virtually no irritating sensation in the oral cavity or pharynx, and retains good dissolution and intraorally rapidly disintegrating properties even when it is stored under high temperature/high humidity conditions.

### [Means for SOLVING THE PROBLEMS]

In view of the above object, the present inventors conducted diligent studies and, as a result, have found that an intraorally rapidly disintegrating tablet comprising Compound I as an active ingredient, when the tablet is made into a preparation containing a particle of Compound I coated with a component containing a methacrylic acid copolymer and further overcoated with a water-soluble saccharide, causes virtually no irritating sensation in the oral cavity or pharynx, and retains good dissolution and oral disintegration properties even when it is stored under high temperature/high humidity conditions.

That is, the present invention is directed to an intraorally rapidly disintegrating tablet comprising a particle containing a 2-(3-cyano-4-isobutyloxyphenyl)-4-methyl-5-thiazolecarboxylic acid-containing core particle coated with a layer containing a methacrylic acid copolymer and further overcoated with a layer containing a water-soluble saccharide.

### [EFFECTS OF THE INVENTION]

According to the present invention, there is provided an intraorally rapidly disintegrating tablet comprising Compound I as an active ingredient and capable of being easily taken. This tablet causes virtually no irritating sensation in the oral cavity or pharynx, and retains good dissolution and intraorally rapidly disintegrating properties even when it is stored under high temperature/high humidity conditions.

### [BRIEF DESCRIPTION OF THE DRAWINGS]

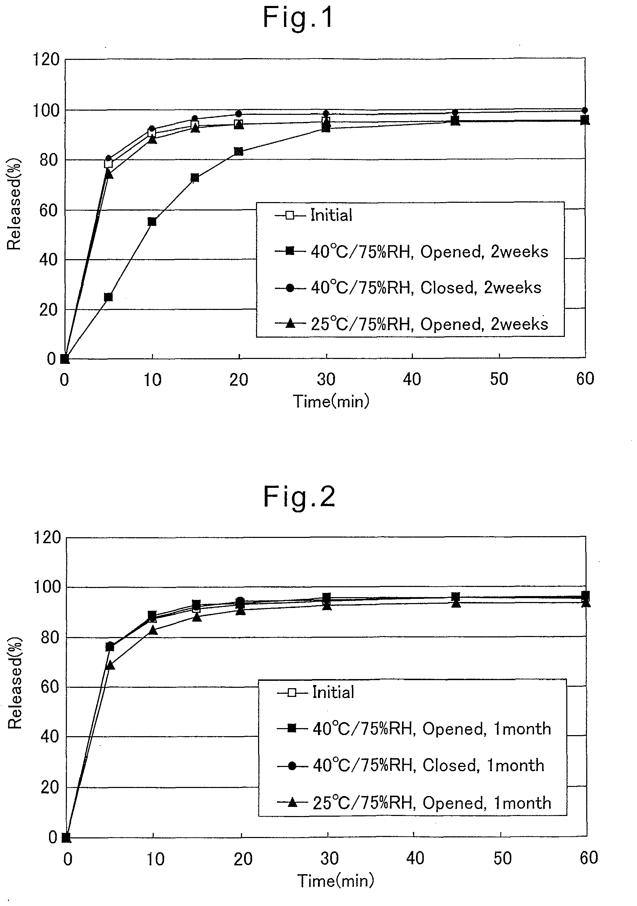

[Figure 1] Figure 1 is a graphical representation showing the initial dissolution properties and dissolution properties after 40°C/75%RH unsealed storage of the tablet of Comparative Example 1.
[Figure 2] Figure 2 is a graphical representation showing the initial dissolution properties and dissolution properties after 40°C/75%RH unsealed storage of the tablet of Example 1.
[Figure 3] Figure 3 is a graphical representation showing the initial dissolution properties and dissolution properties after 40°C/75%RH unsealed storage of the tablet of Example 2.
[Figure 4] Figure 4 is a graphical representation showing the initial dissolution properties and dissolution properties after 40°C/75%RH unsealed storage of the tablet of Reference Example.

### [DESCRIPTION OF THE EMBODIMENTS]

The present invention is directed to an intraorally rapidly disintegrating tablet comprising a particle containing a Compound I-containing core particle coated with a layer comprising a methacrylic acid copolymer and further overcoated with a layer containing a water-soluble saccharide.

In the present invention, "intraorally rapidly disintegrating tablet" means a tablet capable of being taken by a patient, wherein the tablet disintegrates in the oral cavity, within 60 seconds, preferably within 30 seconds, with only saliva in the oral cavity or with a small amount of water. However, it is only necessary for the intraorally rapidly disintegrating properties to be adequate in light of a purpose, and it is not necessary to stick to these values.

The intraorally rapidly disintegrating tablet of the present invention preferably has a practical hardness of 29 N or more, and more preferably of 49 N or more.

The preferred dissolution properties of the tablet of the invention are represented by a 60-min dissolution rate of 80% or more, as measured according to the Japanese Pharmacopoeia Paddle method at 50 revolutions per minute, using a pH 6.0 McIlvaine buffer solution. If the rate is lower than this, there is no comparability in dissolution properties to formulations of Compound I that have guaranteed medicinal effects, and concern about influence on medicinal effects is raised.

The intraorally rapidly disintegrating tablet of the present invention comprises a particle containing a Compound I-containing core particle coated with a coating layer comprising a methacrylic acid copolymer and further overcoated with a water-soluble saccharide (hereinafter the particle is sometimes referred to as "Compound I-containing particle").

The preferred content of Compound I is 5 to 25 wt% of the whole tablet. Specifically, this can be exemplified, for instance, by a 125 mg tablet containing 10 mg or 20 mg of Compound I or a 250 mg tablet containing 40 mg of Compound I. If the content is greater than this, poor masking and intraorally rapidly disintegrating properties may occur, and, furthermore, manufacturability and stability may deteriorate.

Besides Compound I, the core particle in the present invention may contain a fluidizer, such as light anhydrous silicic acid, talc, stearic acid or metal salt thereof, or the like.

The particle diameter of the core particle in the present invention, which is influenced by the particle diameter of Compound I, the existence or kind of fluidizer, and the like, is usually 1 to 50 µm, and preferably 3 to 30 µm, as a median diameter as measured by a laser diffraction technique.

The core particle in the present invention is coated with a layer containing a methacrylic acid copolymer as a main component. Examples of the methacrylic acid copolymer include, but are not particularly limited to, methacrylic acid copolymer LD (for example, trade name: EUDRAGIT L30D55, Evonik), methacrylic acid copolymer L (for example, trade name: EUDRAGIT L100, Evonik), methacrylic acid copolymer S (for example, trade name: EUDRAGIT S100, Evonik), and the like. Aminoalkyl methacrylate copolymers and methacrylic acid ester copolymers, which are enteric polymers as are methacrylic acid copolymers, are not preferable, because, when they are applied to Compound I, poor dissolution and intraorally rapidly disintegrating properties follow, and also larger amounts are required for the same level of masking properties. In addition, ethyl cellulose, which is conventionally used to mask bitterness and the like, is not preferable, because, when it is applied to Compound I, poor dissolution and intraorally rapidly disintegrating properties follow.

The content of the methacrylic acid copolymer is preferably 50 to 99 wt%, and particularly preferably 80 to 95 wt%, of the coating layer.

The coating layer comprising a methacrylic acid copolymer may contain additives that are commonly used in coating agents for enteric formulations or for bitterness masking. Specific examples of such additives may include plasticizers such as polyethylene glycol, propylene glycol, triacetin, triethyl citrate, and the like, anti-adhesives such as talc, stearic acid or metal salt thereof, and the like, and permeability-controlling agents such as polysorbate 80, hydroxypropylcellulose, hydroxypropylmethylcellulose, carboxymethyl cellulose, polyvinyl alcohol, and the like. The amount of plasticizers to be added is usually 1 to 30 wt%, and preferably 1 to 20 wt%, of the whole coating layer, depending on the type of plasticizer. If the amount exceeds 20 wt%, poor dissolution properties may occur after storage, especially after storage under high temperature/high humidity conditions. If the amount is less than 1 wt%, the action as plasticizers will be insufficient.

The content (coating rate) of the coating layer containing a methacrylic acid copolymer, which is influenced by the content or particle diameter of Compound I, and the like, is usually 0 to 60 wt%, and preferably 30 to 60 wt%, relative to the core particle. If the content is 20 wt% or less, insufficient masking properties may occur, and if the content is 70 wt% or more, poor dissolution properties may occur.

In the Compound I-containing particle which has a coating layer containing a methacrylic acid copolymer and further overcoated with a water-soluble polysaccharide, the water-soluble saccharide is not particularly limited as long as it is a water-soluble saccharide used in pharmaceutical formulations. However, the water-soluble saccharide is preferably other than erythritol or sorbitol. For example, one or more water-soluble saccharides selected from the group consisting of mannitol, xylitol, lactitol, palatinit, palatinose, maltitol, maltose, trehalose, lactose, sucrose, glucose, oligosaccharide, fructose, and maltose can be used. Mannitol is particularly preferable.

The content of the overcoating water-soluble saccharide, which is influenced by the kind of water-soluble saccharide used, the content of Compound I, and the like, is usually 1 to 30 wt%, and preferably 5 to 20 wt%, relative to the Compound I-containing particle coated with a layer containing a methacrylic acid copolymer. If the content is less than 1%, poor dissolution of Compound I from formulations or poor disintegration of formulations in the oral cavity will occur after storage, especially after storage under high temperature/high humidity conditions (for example, 40°C, 75% relative humidity). If the content exceeds 30 wt%, the size of formulations is increased, which is not preferable.

The overcoat layer may contain additives that are commonly used in a coating layer, in a range without affecting dissolution and disintegration properties. Specific examples of such additives may include plasticizers such as polyethylene glycol, propylene glycol, triacetin, triethyl citrate, and the like, anti-adhesives such as talc, stearic acid or metal salt thereof, and the like, and permeability-controlling agents such as polysorbate 80, hydroxypropylcellulose, hydroxypropylmethylcellulose, carboxymethyl cellulose, polyvinyl alcohol, and the like. The content of such additives is usually 30 wt% or less for each, and 50 wt% or less in total, relative to the water-soluble saccharide.

The Compound I-containing particle can be produced without difficulty using an ordinary fine particle coating apparatus or a fine particle coating apparatus with some modifications. For example, it can be prepared by charging a fine particle coating apparatus with Compound I along with a fluidizer, and spraying a dispersion containing a methacrylic acid copolymer, subsequently a water-soluble saccharide solution.

By using the Compound I-containing particle instead of active ingredient particles of ordinary intraorally rapidly disintegrating tablet techniques, the intraorally rapidly disintegrating tablet of the present invention can be produced without difficulty using ordinary production equipment or production equipment with some modifications.

An example of preferred technique of intraorally rapidly disintegrating tablet applied to the intraorally rapidly disintegrating tablet of the present invention is the technique disclosed in Japanese Patent Application Publication No. 2009-515871. More specifically, it is a tablet formed by compression molding of: a granule comprising a particle containing an excipient such as mannitol and the like, a fluidizer such as light anhydrous silicic acid and the like, and a disintegrant such as crospovidone and the like, and coated with a disintegrant such as crospovidone and the like; a Compound I-containing particle; and optionally a lubricant such as calcium stearate and the like.

### [EXAMPLES]

### [Comparative Example 1] (Masking with methacrylic acid copolymer coating and no overcoating)

A coating dispersion was prepared by adding 12.6 g of MACROGOL 6000 (NOF CORPORATION, trade name: MACROGOL 6000P) to 650.2 g of purified water to be dissolved, followed by the addition of 420.0 g of methacrylic acid copolymer LD (Evonik, trade name: EUDRAGIT L30D55) and sufficient agitation.

Compound I-containing particles were obtained by charging a fine particle coating/granulating apparatus (POWREX CORPORATION, MP-01SFP) with 300 g of Compound I and 15 g of light anhydrous silicic acid (Freund Corporation), and the resulting mixture was sprayed with the above coating dispersion.

Granules were obtained by charging a fluidized bed granulator (POWREX CORPORATION, MP-01) with 870 g of D-mannitol (TOWAKASEI KOGYO), 40 g of light anhydrous silicic acid (Freund Corporation), and 45 g of crospovidone (ISP), and the resulting mixture was sprayed with purified water. Disintegrant-coated granules were prepared by charging 45 g of crospovidone to powder coat the above granules.

After 35.28 g of Compound I-containing particles and 2.25 g of calcium stearate (NOF CORPORATION) were added to 112.47 g of the disintegrant-coated granules and mixed together, the mixture was subjected to compression molding with a rotary tableting machine (HATA IRON WORKS Co., Ltd., HT-AP6SS-U) to form tablets. The molding condition was a tablet weight of 250 mg, and the tableting was performed using a φ8 mm flat punch with a cleavage line to give a hardness of about 78.5 N.

### [Comparative Example 2] (Masking with hypromellose coating and no overcoating)

A coating dispersion was prepared by adding 100g of hypromellose (Shin-Etsu Chemical Co., Ltd., trade name: Tc-5R) to1900 g of purified water to be dissolved, followed by the addition of 15 g of light anhydrous silicic acid (Nippon Aerosil Co., Ltd., trade name: AEROSIL 200) and sufficient agitation. Subsequently, in the same manner as in Comparative Example 1, Compound I-containing particles were prepared, then mixed with disintegrant-coated granules and calcium stearate (NOF CORPORATION), and tableted to form tablets.

### [Comparative Example 3] (Masking with aminoalkyl methacrylate copolymer coating and no overcoating)

A coating dispersion was prepared by adding 40 g of polysorbate 80 (Wako Pure Chemical Industries), 20 g of sodium carboxymethylcellulose (Dai-ichi Kogyo Seiyaku Co., Ltd., trade name: CELLOGEN PR-S), 40 g of light anhydrous silicic acid (Nippon Aerosil Co., Ltd., trade name: AEROSIL 200), and 60 g of an aminoalkyl methacrylate copolymer (Evonik, trade name: EUDRAGIT RL30D) to 900 g of purified water, followed by sufficient agitation. Subsequently, in the same manner as in Comparative Example 1, Compound I-containing particles were prepared, then mixed with disintegrant-coated granules and calcium stearate (NOF CORPORATION), and tableted to form tablets.

### [Comparative Example 4] (Masking with flavoring agents)

After 24 g of Compound I, 0.75 g of aspartame (Ajinomoto Co., Inc.), 0.15 g of 1-menthol (The Suzuki Menthol Co., Ltd.), and 2.25 g of calcium stearate (NOF CORPORATION), were added to 122.85 g of the disintegrant-coated granules of Comparative Example 1 and mixed together, the mixture was tableted in the same manner as in Comparative Example 1 to form tablets.

### [Test Example 1]

For the tablets of Comparative Examples 1 to 4, oral disintegration time and masking properties were assessed as initial values immediately after preparation. Concerning the assessment method, the assessment was performed by two healthy adult males; after tablets were placed on their tongues and allowed to disintegrate, the properties of masking the irritation of the basis were rated on the following criteria. The results are shown in Table 1.
0: Having a clear masking effect and causing no irritation
1: Having a masking effect and causing little irritation
2: Having a masking effect but causing irritation
3: Having a weak masking effect and causing strong irritation (tolerable)
4: Having no masking effect and causing strong irritation (intolerable)

**[Table 1]**

| | Oral disintegration time (sec) | Masking property |
|---|---|---|
| Comparative Example 1 | 18 | 1 |
| Comparative Example 2 | 48 | 2 |
| Comparative Example 3 | 42 | 2 |
| Comparative Example 4 | 22 | 3 |

According to these results, with respect to the initial values immediately after preparation, only in the case of using the methacrylic acid copolymer of Comparative Example 1, both disintegration and masking properties were good. In the cases of using the hypromellose of Comparative Example 2, and even in the case of using the aminoalkyl methacrylate copolymer of Comparative Example 3, which is an acrylic polymer as is the methacrylic acid copolymer, both disintegration and masking properties were insufficient. In addition, the addition of aspartame and menthol in Comparative Example 4 did not result in adequate masking for Compound I.

### [Example 1] (Masking with methacrylic acid copolymer coating + mannitol coating)

A coating dispersion was prepared by adding 12.6 g of MACROGOL 6000 (NOF CORPORATION, trade name: MACROGOL 6000P) to 650.2 g of purified water to be dissolved, followed by the addition of 420.0 g of methacrylic acid copolymer LD (Evonik, trade name: EUDRAGIT L30D55) and sufficient agitation. Then, an overcoat solution was prepared by adding 70 g of D-mannitol (TOWA-KASEI Co., Ltd.) to 630 g of purified water to be dissolved.

Compound I-containing particles was obtained by charging a fine particle coating/granulating apparatus (POWREX CORPORATION, MP-01SFP) with 300 g of Compound I and 15 g of light anhydrous silicic acid (Freund Corporation), and the resulting mixture was sprayed with 984.0 g of the above coating dispersion, and subsequently with 441.0 g of the overcoat solution.

Subsequently, in the same manner as in Comparative Example 1, Compound I-containing particles, disintegrant-coated granules, and calcium stearate (NOF CORPORATION) were mixed together and tableted to form tablets.

### [Example 2] (With half the amount of MACROGOL 6000 compared with Example 1)

A coating dispersion was prepared by adding 6.3 g of MACROGOL 6000 (NOF CORPORATION, trade name: MACROGOL 6000P) to 607.3 g of purified water to be dissolved, followed by the addition of 420 g of methacrylic acid copolymer LD (Evonik, trade name: EUDRAGIT L30D55) and sufficient agitation. Then, an overcoat solution was prepared by adding 70 g of D-mannitol (TOWA-KASEI Co., Ltd.) to 630 g of purified water to be dissolved.

Compound I-containing particles were obtained by charging a fine particle coating/granulating apparatus (POWREX CORPORATION, MP-01SFP) with 300 g of Compound I and 15.0 g of light anhydrous silicic acid (Freund Corporation), and the resulting mixture was sprayed with 984.0 g of the above coating dispersion, and subsequently 441.0 g of the overcoat solution.

Subsequently, in the same manner as in Comparative Example 1, Compound I-containing particles, disintegrant-coated granules, and calcium stearate (NOF CORPORATION) were mixed together and tableted to form tablets.

### [Test Example 2]

For the tablets of Comparative Example 1 and Examples 1 and 2, oral disintegration time and dissolution properties after unsealed storage at 40°C/75%RH were assessed. In the dissolution test, the assessment was performed according to the Japanese Pharmacopoeia Paddle method at 50 revolutions per minute, using a pH 6.0 McIlvaine buffer solution as a test medium. The results are shown in Table 2. In addition, the masking properties of these formulations were respectively rated 1 according to Test Example 1 above.

**[Table 2]**

| | | Initial value | After 2 weeks | After 1 month |
|---|---|---|---|---|
| Comparative Example 1 | Oral disintegration time (sec) | 19 | 26 | 40 |
| | Hardness (N) | 78.5 | 67.7 | 71.6 |
| Example 1 | Oral disintegration time (sec) | 16 | 22 | 23 |
| | Hardness (N) | 75.5 | 64.7 | 61.8 |
| Example 2 | Oral disintegration time (sec) | 18 | 18 | 19 |
| | Hardness (N) | 78.5 | 66.7 | 58.8 |

The above results showed that, for the tablet containing particles coated only with a methacrylic acid copolymer (Comparative Example 1), disintegration and dissolution were delayed after storage under high temperature/high humidity conditions, though their initial values immediately after preparation were good. However, when the particles in the tablet were further overcoated with mannitol (Example 1), both of the disintegration and dissolution properties after storage under high temperature/high humidity conditions were improved to be good.

As for the coating with a methacrylic acid copolymer, the content of the plasticizer (polyethylene glycol) in the coating layer has an effect on the disintegration properties after storage, and the disintegration properties were better when the content was 4.7 wt% (Example 2) which is lower than 9.3 wt% (Example 1).

### [Reference Example] (Mannitol → erythritol or sorbitol, compared with Example 1)

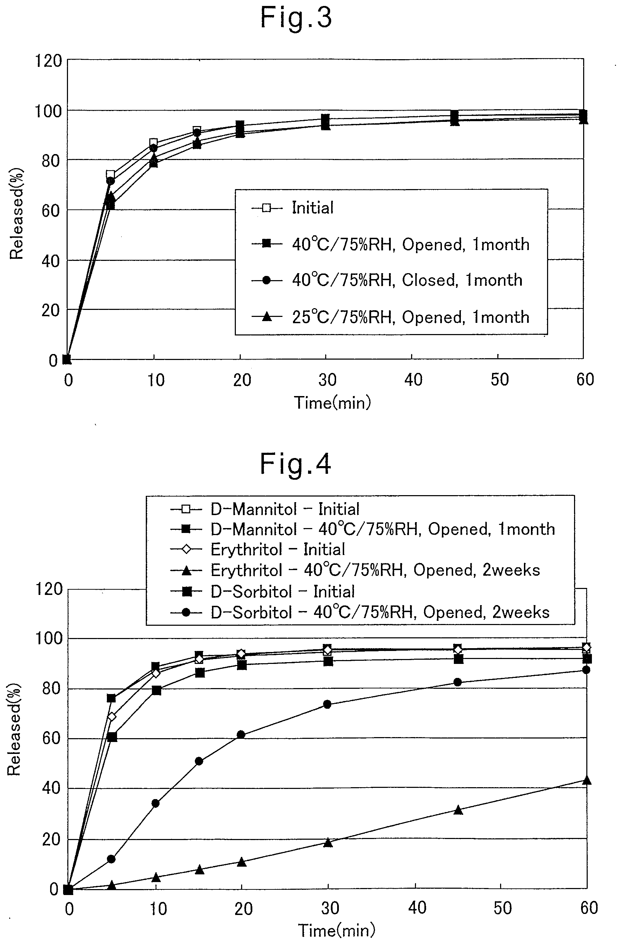

Tablets were formed in the same manner as in Example 1, except that erythritol (NIKKEN CHEMICAL AND SYNTHETIC INDUSTRY Co., Ltd.) or sorbitol (Mitsubishi Shoji Foodtech Co., Ltd., Sorbit DP-50M) was used instead of D-mannitol, and their dissolution properties were assessed in the same manner as in Test Example 2. Results are shown in Figure 4. As shown in Figure 4, the effect of improving dissolution delay after storage under high temperature/high humidity conditions, as was observed in the case of using mannitol, was not observed in the case of using erythritol or sorbitol.

As a result, it has been shown that the intraorally rapidly disintegrating tablet comprising Compound I as an active ingredient, when the tablet is made into a preparation containing a particle of Compound I coated with a layer containing methacrylic acid copolymer and further overcoated with a water-soluble saccharide, causes virtually no irritating sensation in the oral cavity or pharynx, and retains good dissolution and oral disintegration properties even when it is stored under high temperature/high humidity conditions.

### [INDUSTRIAL APPLICABILITY]

The present invention is utilized in preparation of intraorally rapidly disintegrating tablets containing Compound I.

## Claims

1. An intraorally rapidly disintegrating tablet comprising a particle containing a 2-(3-cyano-4-isobutyloxyphenyl)-4-methyl-5-thiazolecarboxylic acid-containing core particle coated with a layer containing a methacrylic acid copolymer and further overcoated with a layer containing a water-soluble saccharide.

2. The tablet of claim 1, wherein the water-soluble saccharide is mannitol.

3. The intraorally rapidly disintegrating tablet according to claim 1, obtained by compression molding of a particle comprising a 2-(3-cyano-4-isobutyloxyphenyl)-4-methyl-5-thiazolecarboxylic acid-containing core particle coated with a layer containing a methacrylic acid copolymer and further overcoated with a layer containing a water-soluble saccharide, and a granule containing a disintegrant-containing particle coated with a disintegrant.

4. The tablet of claim 3, wherein the disintegrant is crospovidone.

## Patentansprüche

1. Tablette mit einer schnellen intraoralen Desintegration, umfassend ein Partikel, welches ein Kernpartikel enthaltend 2-(3-Cyano-4-isobutyl-oxiphenyl)-4-methyl-5-thiazolcarbonsäure umfasst, beschichtet mit einer Lage enthaltend ein Methacrylsäurecoploymer, und ferner überbeschichtet mit einer Lage enthaltend ein wasserlösliches Saccharid.

2. Tablette nach Anspruch 1, wobei das wasserlösliche Sachharid Mannit ist.

3. Tablette mit einer schnellen intraoralen Desintegration nach Anspruch 1, hergestellt durch Formpressen eines Partikels, umfassend ein Kernpartikel enthaltend 2-(3-Cyano-4-isobutyloxiphenyl)-4-methyl-5-thiazolcar-bonsäure, beschichtet mit einer Lage enthaltend ein Methacrylsäurecopolymer, und ferner beschichtet mit einer Lage enthaltend ein wasserlösliches Saccharid, und ein Granulat umfassend ein Sprengmittel enthaltendes Partikel beschichtet mit einem Sprengmittel.

4. Tablette nach Anspruch 3, wobei das Sprengmittel Crospovidon ist.

## Revendications

1. Comprimé à délitement rapide intrabuccal, comprenant une particule contenant une particule de noyau comprenant de l'acide 2-(3-cyano-4-isobutyloxyphényl)-4-méthyl-5-thiazole-carboxylique enrobée d'une couche contenant un copolymère d'acide méthacrylique et recouverte d'un autre enrobage d'une couche contenant un saccharide hydrosoluble.

2. Comprimé selon la revendication 1, dans lequel le saccharide hydrosoluble est le mannitol.

3. Comprimé à délitement rapide intrabuccal selon la revendication 1, obtenu par moulage par compression d'une particule comprenant une particule de noyau comprenant de l'acide 2-(3-cyano-4-isobutyloxyphényl)-4-méthyl-5-thiazole-carboxylique enrobée d'une couche contenant un copolymère d'acide méthacrylique et recouverte d'un autre enrobage d'une couche contenant un saccharide hydrosoluble, et un granulé contenant une particule contenant un agent de désintégration enrobé avec un agent de désintégration.

4. Comprimé selon la revendication 3, dans lequel l'agent de désintégration est la crospovidone.
